(19) Europäisches Patentamt
European Patent Office
Office européen des brevets

(11) **EP 2 483 384 B1**

(12) **FASCICULE DE BREVET EUROPEEN**

(45) Date de publication et mention
de la délivrance du brevet:
**26.02.2014 Bulletin 2014/09**

(51) Int Cl.:
***C12M 1/00*** (2006.01)

(21) Numéro de dépôt: **10765614.2**

(22) Date de dépôt: **01.10.2010**

(86) Numéro de dépôt international:
**PCT/EP2010/064659**

(87) Numéro de publication internationale:
**WO 2011/039354 (07.04.2011 Gazette 2011/14)**

(54) **Photo-bioréacteur couche mince à haute productivité volumique**

Dünnschicht-Photobioreaktor mit höher Produktivität

Thin-layer photobioreactor with high volume productivity

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **01.10.2009 FR 0956870**

(43) Date de publication de la demande:
**08.08.2012 Bulletin 2012/32**

(73) Titulaires:
• **Centre National de la Recherche Scientifique
(C.N.R.S.)
75016 Paris (FR)**
• **Université de Nantes
44000 Nantes (FR)**

(72) Inventeurs:
• **PRUVOST, Jérémy
F-44250 Saint-Brevin Les Pins (FR)**
• **LEGRAND, Jack
F-44600 Saint Nazaire (FR)**
• **LE BORGNE, François
F-53600 Pontivy (FR)**

(74) Mandataire: **Regimbeau
20, rue de Chazelles
75847 Paris Cedex 17 (FR)**

(56) Documents cités:
WO-A2-01/04263    CZ-B6- 279 579
FR-A1- 2 596 412    JP-A- 7 289 239
US-A- 4 253 271    US-A- 5 981 271

**Description**

Domaine de l'invention

L'invention concerne le domaine de la culture cellulaire en photo-bioréacteur. Plus particulièrement, l'invention concerne un photo-bioréacteur pour la culture de cellules comprenant une surface inclinée d'une pente moyenne suivant une direction d'inclinaison sur laquelle ruisselle une solution et présentant un côté amont et un côté aval, la solution comprenant des cellules en suspension.

État de la technique

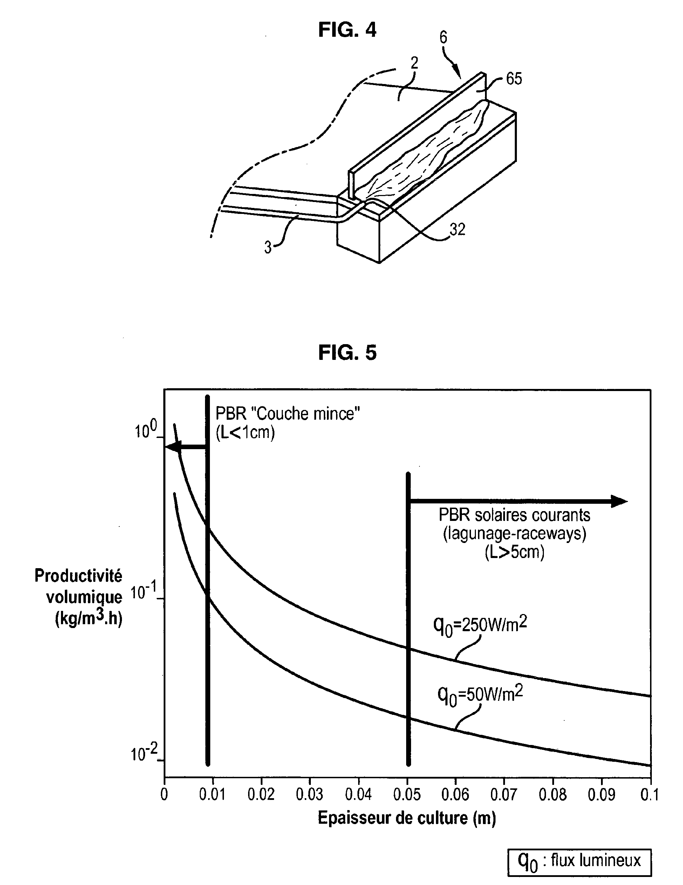

La productivité des photo-bioréacteurs (production de biomasse par unité de volume) est directement liée à la surface spécifique de celui-ci (rapport surface éclairée sur volume de culture). L'apport de lumière est en effet le facteur limitant principal de ce type de procédé. L'absorption de la lumière est importante dans le volume de culture. Il est donc nécessaire d'utiliser des photo-bioréacteurs ayant de grandes surfaces spécifiques éclairées comme le montre la figure 5 représentant la productivité volumique (en kg/m$^3$.h) en fonction de l'épaisseur de la couche de solution (en m) pour deux flux lumineux reçus ($q_0$ = 250 W.m$^{-2}$ et $q_0$ = 50 W.m$^{-2}$).

Les photo-bioréacteurs courants fonctionnent avec des couches de solution d'épaisseur comprise entre 5 cm et 30 cm pour une productivité volumique de 4.10$^{-2}$ kg.m$^{-3}$.h$^{-1}$ maximum (pour une épaisseur de 5 cm et un flux lumineux de 250 W.m$^{-2}$).

Cependant, la recherche pour l'obtention de couche mince mène à un confinement élevé des cultures, engendrant de nombreux problèmes techniques de mise en oeuvre : difficulté d'agiter le milieu pour garantir des transferts suffisants (de nutriments, rapport gaz/liquide, thermiques) et formation de biofilms sur les parois exposées à la lumière empêchant ainsi la lumière de pénétrer dans la solution.

Une solution pour obtenir de grandes surfaces spécifiques éclairées est de diminuer l'épaisseur des solutions dans lesquelles est cultivée la biomasse.

US 5 981 271 donne un exemple de photo-bioréacteur pour la culture d'algues et cyanobactéries, dans lequel l'épaisseur de culture (qui selon les auteurs est habituellement comprise entre 15 cm et 30 cm) est diminuée jusqu'à être comprise entre 5 mm et 18 mm. Ceci est rendu possible en utilisant deux plans rectangulaires inclinés, comprenant chacun un côté largeur amont plus élevé que l'autre côté largeur aval. Le côté largeur aval d'un premier plan étant positionné à la même hauteur que le côté amont d'un deuxième plan et ces deux côtés sont en communication de fluide entre eux. Les deux plans sont disposés tête-bêche, de sorte que le côté aval du deuxième plan se trouve à la verticale du côté amont du premier plan. Un collecteur est placé sous le côté aval du deuxième plan. Un circuit de remontée comprenant une pompe permet d'amener la solution recueillie dans le collecteur vers le côté amont du premier plan. Ainsi la solution ruisselle sur les deux plans et remonte au premier dans un circuit de circulation fermé.

Les deux plans ont des pentes de 1,1 % à 2,5 %, ce qui correspondant à des inclinaisons en angle comprises entre 0,63° et 1,43°.

Un inconvénient de ce photo-bioréacteur est que le collecteur et le circuit de remontée constituent des volumes sombres dans lesquels, les cellules ne sont pas exposées à la lumière. En effet, pour ce photo-bioréacteur, un volume minimal doit être présent dans le collecteur pour que la pompe du circuit de remontée puisse fonctionner. Ces zones sombres ont alors pour conséquence de diminuer la production de biomasse. Aussi les cellules, une fois dans le collecteur ne circulent pas pendant un certain temps, ce qui est néfaste pour la productivité.

Aussi, l'utilisation d'une pompe pour remonter la solution en haut du photo-bioréacteur empêche d'augmenter l'inclinaison. En effet, l'augmentation de l'angle d'inclinaison entraine le besoin d'un collecteur plus important et donc l'augmentation des volumes sombres.

Un autre inconvénient de ce photo-bioréacteur est la disposition de deux supports inclinés tête-bêche nécessaire afin de minimiser le temps passé dans le circuit de remontée. L'utilisation optimale en condition solaire d'éclairement nécessite de maximiser la captation du flux lumineux par le système en recherchant une orientation (par rapport au nord) et une inclinaison (par rapport à l'horizontale) adéquates des supports inclinés. La disposition tête-bêche avec une inclinaison opposée des deux supports inclinés les empêche donc de fonctionner à l'optimum simultanément : l'exposition optimale étant à choisir pour l'une ou l'autre des surfaces. Pour permettre l'écoulement de la culture, l'un des deux supports inclinés est de plus placé à une hauteur plus importante que l'autre. Il en résulte qu'à une période de la journée, ce support incliné crée de l'ombre à la surface du second, diminuant l'efficacité globale du photo-bioréacteur.

FR 2 596 412 donne un autre exemple de photo-bioréacteur comprenant un conduit, longitudinal, ayant une section rectangulaire et dans lequel circule une solution nutritive chargée de micro-organismes. Ce conduit a une position inclinée de sorte que la suspension cellulaire s'écoule par gravité. Le bas du conduit incliné est relié par des canalisations à des moyens de recyclage de telle sorte que la solution nutritive peut remonter du bas vers le haut du conduit incliné. Les moyens de recyclage comprennent un réservoir de stockage dans lequel circule un flux d'air permettant à la solution nutritive de remonter du bas vers le haut du conduit.

Présentation de l'invention

Un but de l'invention est de palier au moins un inconvénient de l'état de la technique. Pour ce faire, l'invention propose un photo-bioréacteur pour la culture de cellules comprenant une surface inclinée d'une pente moyenne suivant une direction d'inclinaison sur laquelle ruisselle une solution et présentant un côté amont et un côté aval, la solution comprenant des cellules en suspension, caractérisé en ce que le photo-bioréacteur comprend une remontée acheminant la solution du côté aval au côté amont suivant une direction de remontée sensiblement parallèle à la direction d'inclinaison.

Un avantage de l'invention est de diminuer le volume sombre et de permettre l'augmentation de l'inclinaison du plan.

D'autres caractéristiques optionnelles et non limitatives sont :

- la remontée est un tube transparent s'étendant parallèlement à la direction d'inclinaison ;
- le photo-bioréacteur comprend un ajusteur de la pente moyenne et la remontée est solidaire du support de ruissellement incliné ;
- le photo-bioréacteur comprend un dispositif de rotation du photo-bioréacteur suivant un axe vertical ;
- le support de ruissellement incliné comprend un point d'hauteur minimale, au niveau duquel est placé un orifice d'entrée de la remontée et au moins un point d'hauteur maximale, au niveau duquel est placé un orifice de sortie de la remontée ;
- le support de ruissellement est plan et rectangulaire, et dans lequel une gouttière est formée par une zone au niveau du côté aval du plan, cette gouttière étant inclinée suivant une direction perpendiculaire à la direction d'inclinaison du support de ruissellement ;
- le photo-bioréacteur comprend un couvercle transparent pour couvrir la surface inclinée, ce couvercle étant adapté pour laisser une couche d'air à la surface de la solution ruisselante ;
- la surface inclinée du support de ruissellement présente des aspérités ;
- un revêtement est appliqué à la surface inclinée du support de ruissellement, le revêtement étant hydrophile pour augmenter l'énergie de surface du support de ruissellement et/ou anti-adhérent pour éviter l'adhésion des cellules sur la surface du support de ruissellement ; et
- un distributeur de la solution est positionné en sortie de la remontée, ce distributeur est adapté pour répartir la solution de manière à ce que le ruissellement recouvre totalement la surface du support de ruissellement.

Présentation des figures

D'autres buts, caractéristiques et avantages apparaîtront à la lecture de la description détaillée ci-après, en référence aux dessins donnés à titre illustratif et non limitatif, parmi lesquels :

- la figure 1 est une représentation d'un exemple de réalisation du photo-bioréacteur selon l'invention ;

- la figure 2 est une représentation partielle d'un exemple de réalisation du photo-bioréacteur selon l'invention avec un tube percé régulièrement comme distributeur de la solution en aval ;
- la figure 3 est une représentation partielle d'une variante du photo-bioréacteur de la figure 2 avec une barre verticale comme distributeur ;
- la figure 4 est une autre vue partielle du photo-bioréacteur de la figure 3 ;
- la figure 5 est un graphe représentant la productivité volumique en fonction de l'épaisseur de la couche de solution ; et
- la figure 6 est un graphe représentant le rendement et le flux transmis à travers la solution contenant des cellules en fonction du rapport temps passé dans le circuit de remontée sur temps passé sur le support de ruissellement.

Description détaillée de l'invention

*Photo-bioréacteur*

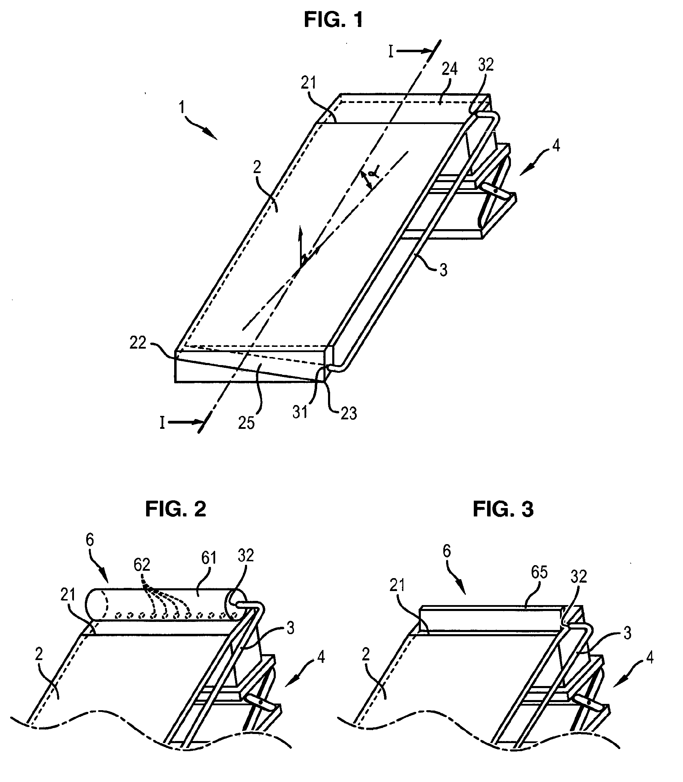

Un exemple de photo-bioréacteur est décrit ci-après en référence à la figure 1.

Le photo-bioréacteur **1** selon l'invention est un photo-bioréacteur pour la culture de cellules en suspension dans une solution, et notamment pour la culture et production de biomasse de micro-algues ou cyanobactéries nécessitant un apport de lumière pour leur photosynthèse.

Le photo-bioréacteur **1** comprend un support de ruissellement **2** sur laquelle ruisselle une solution présentant une surface **20** et incliné d'une pente moyenne suivant une direction d'inclinaison **I-I**. Le support de ruissellement **2** présente un côté amont **21** et un côté aval **22**.

L'inclinaison du support de ruissellement **2** permet la culture en couche mince : la solution ruisselant sur le support de ruissellement **2** en formant un film de faible épaisseur inférieure à 1,5 cm. La culture en couche mince a pour avantage d'augmenter la surface spécifique (rapport de la surface éclairée sur le volume de culture présent sur le support de ruissellement **2**) du photo-bioréacteur **1** et ainsi améliorer le rendement de production volumique de biomasse et atteindre une forte concentration en biomasse supérieure à 10 g/L par rapport aux technologies usuelles (gain d'un rapport 10 environ).

En effet, plus la surface spécifique augmente, et plus la surface éclairée augmente pour un volume donnée. Les réactions de photosynthèse sont alors stimulées dans les micro-algues et cyanobactéries.

Le photo-bioréacteur **1** comprend une remontée **3** qui achemine la solution du côté aval **22** au côté amont **21** suivant une direction de remontée sensiblement parallèle à la direction d'inclinaison **I-I**.

Une telle remontée **3** permet de s'affranchir d'une collecte en aval du ruissellement et donc de supprimer le volume sombre correspondant à celui de la collecte, assurant ainsi une mise en mouvement permanente de la solution contenant les cellules.

Également, une telle remontée **3** parallèle à la direction d'inclinaison **I-I** est plus avantageuse qu'une remontée verticale. En effet, une remontée verticale ne peut être éclairée de manière optimale, puisque cette remontée n'est pas perpendiculaire aux rayons lumineux éclairant le photo-bioréacteur (les rayons lumineux sont de préférence perpendiculaires à la direction d'inclinaison **I-I**). Au contraire, une remontée **3** parallèle à la direction d'inclinaison permet un éclairement optimal de celle-ci en même temps qu'un éclairement optimal du support de ruissellement **2**.

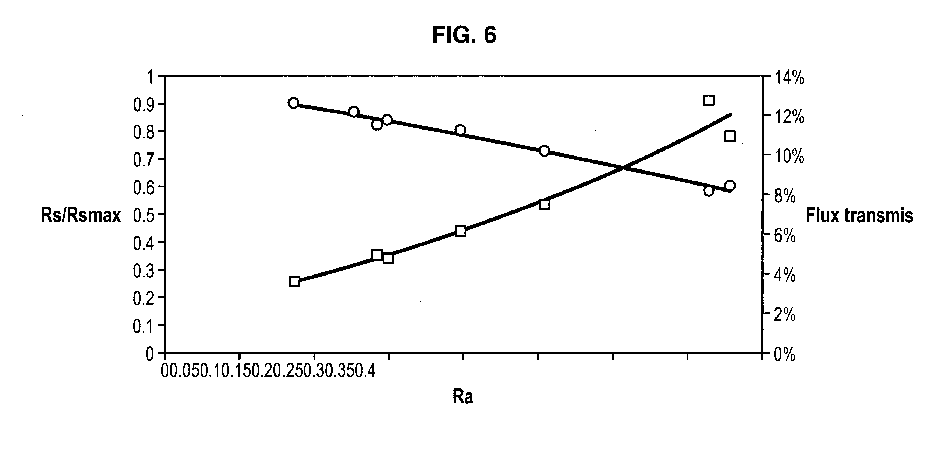

Un facteur important à prendre en compte pour optimiser le rendement en production de biomasse est le ratio $R_a$ entre le temps $T_P$ passé dans la partie en ruissellement (sur le support de ruissellement **2**) et le temps $T_A$ passé dans la partie en remontée (à travers la remontée **3**) $R_a = \dfrac{T_A}{T_P}$ comme illustré sur la figure 6 représentant le rendement en fonction de $R_a$. Ainsi, on remarque que plus $R_a$ augmente et plus le flux lumineux transmis augmente et plus la productivité diminue.

Les valeurs faibles de $R_a$ inférieures à 0,25 garantissent un fonctionnement acceptable du photo-bioréacteur. Ceci implique que le temps passé par les cellules dans la remontée **3** doit être faible par rapport au temps passé par les cellules sur le support de ruissellement **2**. Une remontée **2** parallèle au support de ruissellement **2** répond à cette exigence.

Ceci permet également de s'affranchir de la disposition tête-bêche de l'état de la technique. Le photo-bioréacteur peut alors être orienté de manière à ce que l'inclinaison soit orientée Nord-Sud, ce qui correspond à une exposition optimale.

La remontée **3** peut être un tube transparent s'étendant parallèlement à la direction d'inclinaison **I-I.** L'utilisation d'un tube transparent permet de supprimer le volume sombre correspondant au circuit de remontée de l'état de la technique.

La solution contenant les cellules est mise en mouvement ascendant le long du tube par injection de gaz à l'intérieur du tube ou par un système mécanique de mise en circulation d'un fluide (telle qu'une pompe).

Le tube a de préférence un diamètre inférieur à 1,5 cm afin de ne pas engendrer de volume sombre en son sein. Les cellules sont en suspension dans une solution. Si le diamètre est trop important, les cellules proches des parois du tube peuvent empêcher la lumière d'atteindre celles situées plus à l'intérieur du tube.

La remontée **3** peut être solidaire du support de ruissellement **2** incliné, facilitant ainsi l'utilisation du photo-bioréacteur (il n'y a pas de montage à faire), et assure une étanchéité entre la remontée **3** et le support de ruissellement **2**.

Il est aussi important de choisir un matériau ayant une mouillabilité suffisante caractérisée par une énergie de surface supérieure à 50 mN.m$^{-1}$ pour le support de ruissellement **2,** garantissant ainsi la formation d'un film liquide homogène sur la surface du support de ruissellement **2**. Des exemples de matériaux adéquats sont l'inox et le verre (énergie de surface supérieure à 50 mN.m$^{-1}$).

Le photo-bioréacteur **1** peut comprendre un ajusteur **4** de la pente moyenne. Ceci permet d'optimiser le rendement de production de biomasse sur une année. En effet, au cours d'une année, le zénith du soleil (position journalière du soleil la plus élevée à l'horizon) évolue. Or, l'énergie provenant de la lumière solaire reçue par les cellules dépend de l'angle entre les rayons du soleil et l'inclinaison du support (dans le cas où le photo-bioréacteur est disposé de manière à ce que l'inclinaison soit orientée Nord-Sud). Ainsi, si l'inclinaison ne peut être modifiée, le rendement de production de biomasse serait optimum en été et minimum en hiver. Avec un ajusteur **4** de la pente moyenne, le rendement de production de biomasse peut être optimisé le long de l'année.

L'ajusteur **4** de la pente moyenne peut être manuel ou électrique. Dans le cas où l'ajusteur **4** de la pente moyenne est électrique, il peut être automatisé ou non.

L'ajusteur **4** de la pente moyenne peut être un élévateur mécanique (par exemple à crémaillère ou manivelle) ou hydraulique (piston hydraulique ou pneumatique).

Le photo-bioréacteur **1** peut également comprendre un dispositif de rotation du photo-bioréacteur suivant un axe vertical. Un tel dispositif de rotation du photo-bioréacteur permet d'ajuster l'orientation de l'inclinaison le long de la course du soleil dans le ciel. Ainsi, le rendement de production de biomasse peut être optimisé le long de la journée.

Le dispositif de rotation du photo-bioréacteur peut être manuel ou électrique. Dans le cas où le dispositif de rotation du photo-bioréacteur est électrique, il peut être automatisé ou non.

Le dispositif de rotation du photo-bioréacteur peut être un suiveur de soleil (aussi appelé tracker solaire ou *solar tracker* en anglais). Ce dispositif de rotation peut être tout autre dispositif permettant de suivre la course du soleil dans le ciel, tel un héliostat.

Bien que l'accent ait été mis sur la possibilité de suivre les évolutions du zénith du soleil et de sa course à l'horizon, le photo-bioréacteur selon l'invention peut tout aussi bien être utilisé avec un éclairage naturel ou artificiel.

Le support de ruissellement incliné **2** comprend une zone **23** d'hauteur minimale. Un orifice d'entrée **31** de la remontée **3** est positionné au niveau de la zone **23** d'hauteur minimale.

Ceci permet de diminuer au maximum les zones tampons où la solution s'accumule.

Le support de ruissellement incliné **2** comprend une zone d'hauteur maximale **24**. Un orifice de sortie **32** de la remontée **2** est positionné au niveau de la zone **24** d'hauteur maximale.

Ainsi, une fois la solution contenant les cellules en suspension arrivée à la zone **23** d'hauteur minimale, celle-ci est directement remontée à la zone **24** d'hauteur maximale, minimisant ainsi les parcours inutile de la solution.

En variante, le photo-bioréacteur comprend un support de ruissellement **2** plan et rectangulaire, et dans lequel une gouttière **25** est formée par une zone au niveau du côté aval du plan. La gouttière **25** est inclinée suivant une direction perpendiculaire à la direction d'inclinaison **I-I** du support de ruissellement **2**.

Ainsi, la gouttière **25** permet d'éviter le phénomène de stagnation de la solution du côté aval du support de ruissellement **2** et assure un brassage optimal de la solution contenant les cellules.

La conception d'une gouttière **25** amenant le liquide vers la zone **23** d'hauteur minimale est simple tout en respectant les contraintes majeures au bon fonctionnement du principe d'un photo-bioréacteur à support de ruissellement, à savoir privilégier le temps passé sur la partie en ruissellement par rapport aux autres parties de la boucles hydraulique (remontée **3**, distribution au niveau de la zone d'hauteur maximale, collecte au niveau de la zone d'hauteur minimale).

Aussi, la conception sous forme rectangulaire et plane du support de ruissellement **2** permet une adaptation aisée aux besoins de production en extrapolant par simple multiplication d'unités.

Le photo-bioréacteur **1** peut comprendre un couvercle transparent pour couvrir la surface inclinée. Le couvercle est adapté pour laisser une couche d'air à la surface de la solution ruisselante. Ceci permet d'éviter la formation de biofilms sur la surface éclairée du photo-bioréacteur qui constitue actuellement un inconvénient important de l'état de la technique. De plus, le couvercle permet de diminuer le risque de contamination de la solution par d'autres microorganismes, la régulation thermique et/ou de la phase gazeuse au dessus de la solution ruisselante, ou encore d'empêcher l'évaporation d'eau.

La régulation thermique et/ou de la composition de la phase gazeuse est rendue possible grâce à un régulateur thermique et/ou de gaz connu de l'homme du métier et qui ne sera pas décrit plus en détail par la suite.

La régulation de la phase gazeuse permet de garantir une teneur en carbone dissout suffisante pour éviter une limitation de la croissance photosynthétique des cellules.

Cependant, dans certaines situations, il y a dès lors un risque d'accumulation d'oxygène dans la phase liquide : une trop forte concentration d'oxygène dans la phase liquide peut être toxique pour les micro-algues ou cyanobactéries. Cet oxygène est produit pendant la photosynthèse. Un dispositif de balayage d'air peut alors être prévu pour évacuer le trop plein d'oxygène dans l'atmosphère confinée entre le couvercle et la surface de la solution.

La surface du support de ruissellement **2** peut être lisse ou non. L'avantage d'une surface présentant de micro-aspérités est de permettre un ruissellement légèrement turbulent favorisant les échanges entre la solution contenant les cellules et l'atmosphère située au-dessus de la surface de la solution. Ce ruissellement légèrement turbulent favorise également le renouvellement des cellules puisqu'il y a un brassage léger des cellules. Ainsi, les cellules oscillent entre une position proche de la surface de la solution et une position au fond de la solution, proche de la surface du support de ruissellement **2**.

Un revêtement peut également être appliqué à la surface du support de ruissellement **2**. Ce revêtement peut être un revêtement hydrophile afin d'augmenter l'énergie de surface du support de ruissellement **2**. Ce revêtement peut également être un revêtement anti-adhérent pour empêcher l'adhésion des cellules au support de ruissellement **2,** par exemple un revêtement en un polymère, comme un polymère thermoplastique de tétrafluoroéthylène (PTFE), ayant ou non, subi un traitement hydrophile. Le revêtement peut être un revêtement à la fois hydrophile et anti-adhérent.

Un distributeur **6** de la solution est positionné en sortie de la remontée **3**. Ce distributeur **6** est adapté pour répartir la solution de manière à ce que le ruissellement recouvre totalement la surface du support de ruissellement **2**. Ceci assure une utilisation optimale de la surface du support de ruissellement **2**.

Un exemple dans le cas où le support de ruissellement **2** est rectangulaire avec deux côtés largeur et deux côtés longueur est donnée ci-après. La direction d'inclinaison **I-I** est sensiblement parallèle aux côtés longueur. Le distributeur est adapté pour répartir la solution de manière à recouvrir totalement la surface du support de ruissellement **2** sur son côté largeur correspondant au côté amont **21**.

Ce distributeur **6** peut être un tube **61** percé régulièrement d'orifices **62** et s'étendant parallèlement au côté amont **21** comme illustré sur la figure 2.

Ce distributeur **6** peut aussi être une barre verticale **65** ajustable en hauteur disposé au niveau du côté amont **21** et perpendiculaire au support de ruissellement **2** à la manière d'une sous-verse, comme illustré sur les figures 3 et 4. La barre verticale **65** est positionnée légèrement au dessus de la surface du support de ruissellement **65**. Ainsi, quand la solution arrive par l'orifice de sortie **32** de la remontée **3,** celle-ci se répand en amont de la barre verticale **65**. La barre verticale **65** retient une partie de la solution : la solution se répand alors le long de la barre verticale **65** et éventuellement vers l'extrémité côté amont du support de ruissellement **2**.

Pour de forte inclinaison du support de ruissellement **2,** c'est-à-dire pour des inclinaisons supérieures à 5°, les deux types de distributeurs mentionnés ci-dessus peuvent être avantageusement combinés. Dans ce cas, le tube percé est positionné en amont de la barre verticale ; le tube percé permettant une première répartition de la solution au niveau du côté amont du support de ruissellement **2** et la barre verticale permet par une faible rétention d'homogénéiser le flux. On obtient alors un film ruisselant homogène.

Le photo-bioréacteur **1** selon l'invention permet un fonctionnement à un point optimal en ce qui concerne la conversion du flux lumineux capté sans limitation due au taux de carbone ou nutriments.

Le rendement de production de biomasse gagne un ordre de grandeur par rapport aux photo-bioréacteurs conventionnels.

Ainsi, lors de test, les présents auteurs ont constaté une productivité maximale de $20 \cdot 10^{-2}$ kg.m$^{-3}$h$^{-1}$, soit une concentration en biomasse de 17 kg.m$^{-3}$ à l'optimum de fonctionnement du photo-bioréacteur selon l'invention (contre 1 à 5 kg.m$^{-3}$ pour les photo-bioréacteurs conventionnels) (pour un éclairement de 250 W.m$^{-2}$).

*Dimensions*

Un exemple de dimensionnement est donné ci-après.

Dans cet exemple, le photo-bioréacteur **1** possède un support de ruissellement **2** rectangulaire avec des côtés largeur de 70 cm et des côtés longueur de 100 cm et une remontée **3** sous forme de tube transparent à section circulaire d' 1 cm de diamètre. L'inclinaison du support de ruissellement **2** est de 5°.

Bien entendu les dimensionnements sont donnés ici simplement à titre illustratif et ceux-ci peuvent être modifiés selon les besoins de production. Par exemple, la largueur du support de ruissellement **2** peut être réduite jusqu'à 30 cm, le diamètre du tube augmenté jusqu'à 1,5 cm et l'inclinaison augmentée jusqu'à 15°.

*Application*

Bien que l'invention soit décrite en référence à son application à la production de biomasse, elle peut également être utilisée dans le domaine de la photocatalyse pour le traitement de liquide. En effet, dans ce domaine, une surface spécifique éclairée important est également nécessaire (besoin d'éviter l'encrassement des parois éclairées, de transfert de matière élevé...).

**Revendications**

1. Photo-bioréacteur (1) pour la culture de cellules comprenant un support de ruissellement (2) présentant une surface et incliné d'une pente moyenne suivant une direction d'inclinaison (I-I) sur laquelle ruisselle une solution et présentant un côté amont (21) et un côté aval (22), la solution comprenant des cellules en suspension, **caractérisé en ce que** le photo-bioréacteur (1) comprend une remontée (3) acheminant la solution du côté aval (22) au côté amont (21) suivant une direction de remontée sensiblement parallèle à la direction d'inclinaison (I-I).

2. Photo-bioréacteur (1) selon la revendication 1, dans lequel la remontée (3) est un tube transparent s'étendant parallèlement à la direction d'inclinaison (I-I).

3. Photo-bioréacteur (1) selon la revendication 1 ou 2, comprenant un ajusteur (4) de la pente moyenne et dans lequel la remontée (3) est solidaire du support de ruissellement (2) incliné. ,

4. Photo-bioréacteur selon l'une des revendications 1 à 3, comprenant un dispositif de rotation du photo-bioréacteur suivant un axe vertical.

5. Photo-bioréacteur selon l'une des revendications 1 à 4, dans lequel le support de ruissellement incliné (2) comprend un point (23) d'hauteur minimale, au niveau duquel est placé un orifice d'entrée (31) de la remontée (3) et au moins un point d'hauteur maximale (24), au niveau duquel est placé un orifice de sortie (32) de la remontée (3).

6. Photo-bioréacteur (1) selon la revendication 5, dans lequel le support de ruissellement (2) est plan et rectangulaire, et dans lequel une gouttière (25) est formée par une zone au niveau du côté aval du plan, cette gouttière (25) étant inclinée suivant une direction perpendiculaire à la direction d'inclinaison (I-I) du support de ruissellement.

7. Photo-bioréacteur (1) selon l'une des revendications 1 à 6, comprenant un couvercle transparent pour couvrir la surface inclinée, ce couvercle étant adapté pour laisser une couche d'air à la surface de la solution ruisselante.

8. Photo-bioréacteur (1) selon l'une des revendications 1 à 7, dans lequel la surface inclinée du support de ruissellement (2) présente des aspérités.

9. Photo-bioréacteur (1) selon l'une des revendications 1 à 8, dans lequel est appliqué à la surface inclinée du support de ruissellement (2) un revêtement hydrophile pour augmenter l'énergie de surface du support de ruissellement (2) et/ou anti-adhérent pour éviter l'adhésion des cellules sur la surface du support de ruissellement (2).

10. Photo-bioréacteur (1) selon l'une des revendications 1 à 9, dans lequel un distributeur (6) de la solution est positionné en sortie de la remontée (3), ce distributeur (6) est adapté pour répartir la solution de manière à ce que le ruissellement recouvre totalement la surface du support de ruissellement (2).

**Patentansprüche**

1. Photobioreaktor (1) für die Züchtung von Zellen, der ein Berieselungsgestell (2) umfasst, das eine Fläche aufweist, die mit einer mittleren Steigung in einer Neigungsrichtung (I-I) geneigt ist und über die eine Lösung läuft, und das eine flussaufwärts liegende Seite (21) und eine flussabwärts liegende Seite (22) aufweist, wobei die Lösung Zellen in Suspension umfasst, **dadurch gekennzeichnet, dass** der Photobioreaktor (1) ein aufsteigendes Rückführungselement (3) umfasst, das die Lösung von der flussabwärts liegenden Seite (22) zur flussaufwärts liegenden Seite (21) führt, und dies in einer aufsteigenden Rückführungsrichtung, die im Wesentlichen parallel zur Neigungsrichtung (I-I) verläuft.

2. Photobioreaktor (1) nach Anspruch 1, bei dem das aufsteigende Rückführungselement (3) eine durchsichtige Röhre ist, die sich parallel zur Neigungsrichtung (I-I) erstreckt.

3. Photobioreaktor (1) nach Anspruch 1 oder 2, der eine Einstellvorrichtung (4) für die mittlere Steigung umfasst und bei dem das aufsteigende Rückführungselement (3) fest mit dem geneigten Berieselungsgestell (2) verbunden ist.

4. Photobioreaktor (1) nach einem der Ansprüche 1 bis 3, der eine Vorrichtung zum Drehen des Photobioreaktors um eine vertikale Achse umfasst.

5. Photobioreaktor (1) nach einem der Ansprüche 1 bis 4, bei dem das geneigte Berieselungsgestell (2) Folgendes umfasst: einen Punkt (23) mit minimaler Höhe, auf dessen Höhe eine Eintrittsöffnung (31) des aufsteigenden Rückführungselement (3) angeordnet ist, und wenigstens einen Punkt maximaler Höhe (24), auf dessen Höhe eine Austrittsöffnung (32) des aufsteigenden Rückführungselement (3) angeordnet ist.

6. Photobioreaktor (1) nach Anspruch 5, bei dem das Berieselungsgestell (2) eben und rechteckig ist und bei dem durch eine auf Höhe der flussabwärts liegenden Seite der Ebene befindliche Zone eine Rinne (25) gebildet wird, wobei diese Rinne (25) in einer Richtung senkrecht zur Neigungsrichtung (I-I) des Berieselungsgestells geneigt ist.

7. Photobioreaktor (1) nach einem der Ansprüche 1 bis 6, der zum Abdecken der geneigten Fläche einen durchsichtigen Deckel umfasst, wobei der Deckel dafür eingerichtet ist, an der Fläche der rieselnden Lösung eine Luftschicht zu lassen.

8. Photobioreaktor (1) nach einem der Ansprüche 1 bis 7, bei dem die geneigte Fläche des Berieselungsgestells (2) Unebenheiten aufweist.

9. Photobioreaktor (1) nach einem der Ansprüche 1 bis 8, bei dem auf die geneigte Fläche des Berieselungsgestells (2) eine hydrophile Beschichtung zum Erhöhen der Oberflächenenergie des Berieselungsgestells (2) und/oder eine Antihaft-Beschichtung zum Verhindern des Anhaftens der Zellen auf der Fläche des Berieselungsgestells (2) angewendet ist.

10. Photobioreaktor (1) nach einem der Ansprüche 1 bis 9, bei dem am Ausgang des aufsteigenden Rückführungselements (3) eine Ausgabevorrichtung (6) für die Lösung angeordnet ist, wobei die Ausgabevorrichtung (6) dafür eingerichtet ist, die Lösung derart zu verteilen, dass die Berieselung die Fläche des Berieselungsgestells (2) vollständig bedeckt.

## Claims

1. Photo-bioreactor (1) for the culture of cells comprising a trickle support (2) which presents a surface and inclined with a moderate slope along a direction of inclination (1-1) on which a solution trickles and which presents an upstream side (21) and a downstream side (22), where the solution comprises cells in suspension, **characterised in that** the photo-bioreactor (1) comprises a return pipe (3) which leads the solution from the downstream side (22) to the upstream side (21) along a return direction which is substantially parallel to the direction of inclination (1-1).

2. Photo-bioreactor (1) according to claim 1, wherein the return pipe (3) is a transparent tube extending parallel to the direction of inclination (1-1).

3. Photo-bioreactor (1) according to claim 1 or 2, comprising an adjuster (4) of the moderate slope and wherein the return pipe (3) forms part of the inclined trickle support (2).

4. Photo-bioreactor according to one of claims 1 to 3, comprising a device for rotation of the photo-bioreactor along a vertical axis.

5. Photo-bioreactor according to one of claims 1 to 4, wherein the inclined trickle support (2) comprises a point (23) of minimum height, at which an inlet orifice (31) to the return pipe (3) is located and at least one point of maximum height (24) at which an outlet orifice (32) from the return pipe (3) is located.

6. Photo-bioreactor (1) according to claim 5, wherein the trickle support (2) is flat and rectangular, and wherein a trough (25) is formed by a zone at the downstream side of the plane, with this trough (25) being inclined along a direction perpendicular to the direction of inclination (1-1) of the trickle support.

7. Photo-bioreactor (1) according to one of claims 1 to 6, comprising a transparent cover for covering the inclined surface, this cover being suitable for leaving a layer of air at the surface of the trickling solution.

8. Photo-bioreactor (1) according to one of the claims 1 to 7, wherein the inclined surface of the trickle support (2) exhibits rough areas.

9. Photo-bioreactor (1) according to one of claims 1 to 8, wherein on the inclined surface of the trickle support (2) a hydrophilic coating is applied in order to increase the surface energy of the trickle support (2) and/or an anti-adherent coating is applied to prevent the adhesion of cells onto the surface of the trickle support (2).

10. Photo-bioreactor (1) according to any of claims 1 to 9, wherein a distributor (6) of the solution is placed at the outlet of the return pipe (3), this distributor (6) being suitable for distributing the solution such that the trickle completely covers the surface of the trickle support (2).

# FIG. 1

# FIG. 2

# FIG. 3

## FIG. 4

## FIG. 5

PBR "Couche mince" (L<1cm)

PBR solaires courants (lagunage-raceways) (L>5cm)

$q_0$=250W/m$^2$

$q_0$=50W/m$^2$

Productivité volumique (kg/m$^3$.h)

Epaisseur de culture (m)

$q_0$ : flux lumineux

# FIG. 6

Rs/Rsmax

Flux transmis

Ra

EP 2 483 384 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5981271 A
- FR 2596412